# EUROPÄISCHE PATENTANMELDUNG

(11) **EP 2 201 941 A1**
(43) Veröffentlichungstag der Anmeldung: **30.06.2010**
(21) Anmeldenummer: 08173017.8
(22) Anmeldetag: 29.12.2008
(51) Int. Cl.: A61K 9/70, A61F 13/02, B26F 1/20, B26F 1/38, B26F 3/10

(54) **Pflaster-Herstellungstechnologie**

(71) Anmelder: UCB Pharma GmbH, 40789 Monheim (DE)
(72) Erfinder: Jasch, Ingolf, 49652, Solingen (DE)
(74) Vertreter: HOFFMANN EITLE

(57) **Zusammenfassung**

Die Erfindung betrifft ein transdermales, insbesondere dopaminerges Pflaster, umfassend eine Release-Liner-Folie, eine Wirkstoffschicht und eine Trägerschicht, wobei die Wirkstoffechicht zwischen der Release-Liner-Folie und der Trägerschicht eingebracht ist. Dabei weist die Release-Liner-Folie und/oder die Trägerschicht zumindest eine thermisch bewirkte Trennkante auf, die zumindest teilweise einen umfangseitigen Rand des Pflasters definiert. Ferner betrifft die Erfindung ein Verfahren zur Herstellung eines solchen transdermalen Pflasters sowie ein Werkzeug zum Herstellen des Pflasters gemäß dem erfindungsgemäßen Verfahren. Durch das erfindungsgemäße Pflaster kann ein Kristallwachstum in der Wirkstoffschicht zuverlässig unterbunden werden.

## Beschreibung

### Technisches Gebiet

Die Erfindung betrifft ein transdermales Pflaster mit einer Release-Liner-Folie, einer Wirkstoffschicht mit dem Wirkstoff Rotigotin und/oder einem seiner pharmakologisch akzeptablen Salze und einer Trägerschicht, wobei die Wirkstoffschicht zwischen der Release-Liner-Folie und der Trägerschicht eingebracht ist. Des Weiteren betrifft die Erfindung ein Verfahren zur Herstellung eines transdermalen Pflasters sowie ein Werkzeug zum Herstellen eines solchen Pflasters.

### Hintergrund

Transdermale Pflaster dienen der Applikation von Arzneistoffen durch die Haut. Nach Aufkleben eines transdermalen Pflasters auf die Haut gelangt ein für diese Anwendung geeigneter Arzneistoff kontinuierlich direkt in den systemischen Blutkreislauf, d.h. unter Umgehung des Magen - Darm- Traktes und der ersten Leberpassage, wodurch z.B. Magenunverträglichkeit und/oder der frühzeitige hepatische "first-pass" Effekt (Abbau in der Leber ) bestimmter Substanzen nach oraler Gabe vermieden wird. Beispiele hierfür sind Nikotinpflaster, Hormonpflaster und Schmerzpflaster sowie transdermale Pflaster zur Behandlung der Parkinson-Krankheit oder für die Behandlung der ruhelosen Beine (restless legs). Solche therapeutischen Pflaster können beispielsweise den dopaminergen Wirkstoff Rotigotin enthalten. Er ist unter anderem zur Behandlung der Parkinsonschen Krankheit und zur Therapie der ruhelosen Beine ("restless legs") geeignet und entsprechende transdermale Pflaster werden bereits in einigen Ländern angewendet. Medizinische Verwendungen von Rotigotin bzw. Rotigotin-haltigen Arzneiformen sind beispielsweise beschrieben in WO 2005/92331, WO 2005/009424, WO 2007/147556, WO 03/92677 sowie WO 2005/63237.

Eine aus dem Stand der Technik bekannte und bevorzugte Ausführungsform eines transdermalen Pflasters besteht aus einer Wirkstoffschicht, einer Trägerschicht und einer Release Liner Folie. So aufgebaute Pflaster werden üblicherweise aus einem großflächigen Laminat ausgestanzt.
Nach dem Stanzen ist die Wirkstoffschicht an den umfangseitigen Trennkanten offen, d.h. nicht durch Folien abgedeckt.

Eine mögliche Herstellung von Rotigotinpflastern, die o.g. Ausführungsform entsprechen, wird beispielsweise in der WO 02/089778 und der WO 04/012730 beschrieben. Der Wirkstoff Rotigotin ist dabei in nicht kristalliner Form in einer silikonkleberhaltigen Schicht enthalten. Vor dem Einbringen von Rotigotin in die Kleberschicht wird Rotigotin in einem Lösungsmittel gelöst, die lösungsmittelhaltige, mit Wirkstoff beladene Klebermasse wird in einem nachfolgenden kontinuierlichen Beschichtungsprozeß auf eine Release-Liner-Folie, eine Polyesterfolie, die auch als "Release-Liner" oder "Schutzfolie" bezeichnet wird, aufgebracht und das Lösungsmittel durch Erwärmen in einem Trockenkanal entfernt. Nach dem Trocknungsprozess wird auf die verbleibende offene Grenzfläche der Rotigotin-haltigen Kleberschicht eine für den Wirkstoff undurchlässige Trägerschicht aufkaschiert. Das so hergestellte Laminat wird anschließend durch mechanische Vereinzelung in einzelne Pflaster aufgeteilt.

Die Trennkanten zwischen den einzelnen Pflastern werden herkömmlich durch mechanische Vereinzelung, zum Beispiel durch Schneiden oder Ausstanzen erzeugt. Die Release-Liner-Folien können darüber hinaus häufig mit einem S-förmigen Schnitt, dem so genannten "S-Cut", versehen werden. Dieser Schnitt erleichtert ein Ablösen der Release-Liner-Folie von dem Pflaster, um das Pflaster mit seiner Rotigotin-haltigen Kleberschicht auf die Haut des Patienten aufzukleben. Der S-Cut kann dabei einerseits als entlang des gesamten Pflasters durchgehender Schnitt oder auch als "Sollbruchstelle" in Form einer gezielten Schwächung der Release-Liner-Folie entlang einer S-Cut-Linie verwirklicht werden. Ein mögliches Herstellungsverfahren für ein transdermales Pflaster wird beispielsweise in der WO 04/012730, als Beispiel 1, beginnend auf Seite 14 detailliert beschrieben.

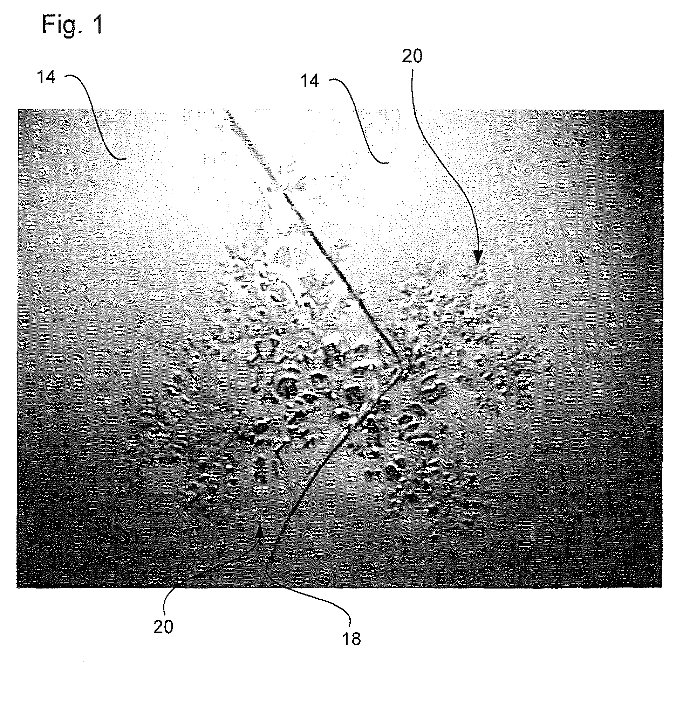

Bei einer Lagerung der so erzeugten Pflaster bei Raumtemperatur besteht jedoch die Gefahr, dass sich Rotigotin-Kristalle in der Wirkstoffschicht im Bereich der Trennkanten ausbilden, und ausgehend von den von den Kanten des Pflasters bzw. dem S-Cut der Release-Liner-Folie in Richtung des Inneren der Wirkstoffschicht, also von der entsprechenden Kante weg, aber auch entlang der Kante ausbreiten. Fig. 1 zeigt eine Trennkante 18 eines Rotigotin-haltigen Pflasters, von der sich Kristalle 20 in die Wirkstoffschicht 14 ausbreiten. Die entstehenden Kristalle bilden ein thermodynamisch stabiles Polymorph II ("Form II") von Rotigotin. Die Bildung eines solchen kristallinen Polymorphs ist in einer Pflasterformulierung unerwünscht; daher wurden bereits Anstrengungen unternommen, eine solche Kristallisation zu vermeiden. Hierzu wird bisher in einigen Fällen eine kontinuierliche Kühlung der Pflaster angewendet, um das Wachstum der Kristalle zu hemmen. Dies erfordert jedoch hohen logistischen und finanziellen Aufwand zur Aufrechterhaltung der Kühlkette von der Herstellung bis zur Anwendung, und ist in einigen Ländern nur schwer umsetzbar. Zudem wird auch die Mitarbeit jedes Patienten benötigt und damit die Handhabung für die Patienten erschwert.

Auch bei Pflastern mit anderen Wirkstoffen stellt eine solche Kristallisation des Wirkstoffs in der Nähe der gegenüber der Umgebung offenen Matrixrändern ein Problem dar. Dies gilt insbesondere für silikonbasierte Pflaster, deren Matrix eine hohe Durchlässigkeit für Wasserdampf und Sauerstoff besitzt, und für lipophile, insbesondere schlecht wasserlösliche Wirkstoffe.

### Darstellung der Erfindung

Die Aufgabe der vorliegenden Erfindung liegt daher darin, ein transdermales Pflaster zu entwickeln, bei dem die Entstehung von Kristallen in der Wirkstoffschicht erschwert und möglichst verhindert wird.

Die Lösung der Aufgabe wird durch ein transdermales Pflaster gegeben, umfassend eine Release-Liner-Folie,
eine Wirkstoffschicht umfassend eine einen schlecht wasserlöslichen Wirkstoff enthaltende Polymerschicht und eine Trägerschicht, wobei die Wirkstoffschicht zwischen der Release-Liner-Folie und der Trägerschicht eingebracht ist, und wobei die Release-Liner-Folie und/oder die Trägerschicht mindestens eine thermisch bewirkte umfangseitige Trennkante aufweisen/aufweist, die zumindest teilweise einen umfangseitigen Rand des Pflasters definiert. In einer bevorzugten Form der Erfindung werden die Release-Liner-Folie und die Trägerschicht dabei über eine Länge von mindestens 70%, bevorzugt über die gesamte Länge der umfangseitigen Trennkanten thermisch und voneinander lösbar aneinander gebunden, insbesondere derart, dass die Wirkstoffschicht dadurch versiegelt ist.

Die Release-Liner-Folie wird bei der Applikation des Pflasters vor dem Aufbringen auf die Haut des Patienten abgezogen und ist daher leicht von der üblicherweise selbst klebenden Wirkstoffschicht ablösbar. Im auf die Wirkstoffschicht aufgebrachten Zustand stellt die Release-Liner-Folie eine Schutzschicht für den Wirkstoff und die diesen enthaltende Wirkstoffschicht dar. Während der Herstellung des Pflasters dient die Release-Liner-Folie aufgrund ihrer physikalischen Eigenschaften (wie z.B. Formstabilität und Reißfestigkeit unter Zugbeanspruchung ) bevorzugt als Träger für die Wirkstoffschicht. Die Release-Liner-Folie stellt auch sicher, dass der Wirkstoff während der Lagerung in einer gewünschten hohen Konzentration in der Wirkstoffschicht verbleibt und sich nicht vor der Anwendung des Pflasters verflüchtigt. Die Release-Liner-Folie nimmt damit sowohl eine Schutzfunktion als auch eine Versiegelungsfunktion für die Wirkstoffschicht wahr und kann vor der Anwendung des Pflasters abgezogen werden.

Die Wirkstoffschicht enthält einen Wirkstoff, der aufgrund seiner physikochemischen Eigenschaften zur transdermalen Verabreichung geeignet ist. Im allgemeinen sind geeignete Wirkstoffkandiaten lipophil und nur begrenzt in Wasser löslich. Nimmt die Wirkstoffschicht bei Lagerung Feuchtigkeit auf, was z.B. in Gegenwart hygroskopischer Hilfstoffe möglich ist, kann die Löslichkeitsgrenze des lipophilen Wirkstoffes im Pflaster überschritten werden, mit der Folge von Kristallkeimbildung und Kristallwachstum. Dieses Risiko ist insbesondere bei lipophilen Wirkstoffen mit schlechter Wasserlöslichkeit ausgeprägt. Unter "schlecht wasserlöslichen Wirkstoffen" werden in dieser Anmeldung Wirkstoffe verstanden, die bei einem pH von 7 bei 15°C eine Wasserlöslichkeit von höchstens 1 mg/mL haben. Beispiele für solche Wirkstoffe sind Estradiol, Buprenorphin, Fentanyl, Norethindroneacetate, und insbesondere Rotigotin und seine Salze. Mit Vorteil liegt der Wirkstoff, z.B. Rotigotin in der Wirkstoffschicht im Wesentlichen in Form der Wirkstoffbase und in 1-20 Gew% vor.

Dabei ist es denkbar, dass die Wirkstoffschicht gleichsam als Klebeschicht für das Pflaster dient. Es ist jedoch auch möglich, dass eine separate Wirkstoffschicht, die keine Klebefunktion wahrnimmt, und eine zusätzliche Klebeschicht vorhanden sind.

Die Trägerschicht kann auch als Rückschicht bezeichnet werden und ist wirkstoffundurchlässig. Die Rückschicht dient in aufgeklebtem Zustand des transdermalen Pflasters dem Schutz des Wirkstoffs vor einer Verflüchtigung und dient zugleich als Trägerschicht für den Wirkstoff, nachdem die Release-Liner-Folie von dem Pflaster abgezogen wurde. Sowohl die Release-Liner-Folie, als auch die Trägerschicht liegen bevorzugt in Folienform vor.

Die Release-Liner-Folie und/oder die Trägerschicht weisen bzw. weist zumindest eine Trennkante auf, die einen umfangseitigen Rand des Pflasters definiert (nachfolgend "umfangseitige Trennkante"), und die thermisch bewirkt ist. In einer bevorzugten Ausführungsform werden mindestens etwa 60%, 70%, 75%, 80%, 90%, 95% oder 99% der Gesamttrennkantenlänge des umfangseitigen Rands des Pflasters thermisch bewirkt. Besonders bevorzugt werden alle umfangsseitigen Trennkanten des Pflasters vollständig thermisch bewirkt.

"Und/oder" bedeutet, dass mindestens eine der Release-Liner-Folie und der Trägerschicht eine thermisch bewirkte Trennkante aufweist. Eine Trennkante im Sinne der Erfindung kann dabei einerseits zur Vereinzelung des Pflasters dienen und somit den umfangseitigen Rand des Pflasters oder Teile davon bilden. Daneben kann als Trennkante auch ein innerhalb des Randes des Pflasters, also auf einer Schicht eines vereinzelten Pflasters vorgenommener teilweiser Schnitt oder eine gezielte Schwächung einer der Schichten verstanden werden (nachfolgend "Sollbruch-Trennkante" oder "Sollbruchkante"). Die Trennkante kann also einerseits eine umfangseitige Kante sein, an der ein Pflaster von einem anderen Pflaster oder einem Rohling getrennt ist, andererseits kann die Trennkante auch eine Sollbruch-Trennkante sein, die zum Auftrennen einer Schicht, z.B. der Release-Liner-Folie, zur Benutzung des Pflasters dient. "Trennen" wird somit als quantitatives Teilen und somit Gegenstück zu "Fügen" verstanden.

Bevorzugt werden die Release-Liner-Folie und die Trägerschicht zumindest entlang einer der umfangseitigen Trennkanten des Pflasters thermisch und voneinander lösbar aneinander gebunden. Bevorzugt wird insbesondere, dass sie derart aneinander gebunden sind, dass die Wirkstoffschicht dadurch versiegelt ist. In einer bevorzugten Ausführungsform sind die Release-Liner-Folie und die Trägerschicht mindestens entlang 60%, 70%, 75%, 80%, 90%, 95% oder 99% der Gesamtlänge der umfangseitigen Trennkanten thermisch und voneinander lösbar aneinander gebunden.

Eine durchgehende "umfangseitige Trennkante" entlang des Umfangs des Pflasters, beispielsweise bei einem kreis- oder ellipsenförmigen Pflaster kann dabei als mehrere Trennkanten angesehen werden, bei denen die Übergänge zwischen den einzelnen Trennkanten kontinuierlich sind.

Besonders bevorzugt sind Release-Liner-Folie und die Trägerschicht über die gesamte Länge der umfangseitigen Trennkanten thermisch und voneinander lösbar aneinander gebunden, so dass die thermische Verbindung der Release-Liner-Folie und der Trägerschicht entlang der umfangseitigen Trennkante die Wirkstoffschicht entlang dieser Trennkante einschließt. In einem solchen Fall kann die Wirkstoffschicht im Wesentlichen gegenüber der Umwelt abgeschlossen und dadurch versiegelt sein. Der Begriff "im Wesentlichen gegenüber der Umwelt abgeschlossen" bedeutet, dass der Gas- und Wirkstoffaustausch zwischen der Wirkstoffschicht und der Pflasterumgebung im Gegensatz zu den aus dem Stand der Technik bekannten Verfahren auch im Kantenbereich in dem Maß unterbunden ist, wie es sich aus den Materialeigenschaften der Träger - und Release Liner Folien ergibt. Üblicherweise werden Träger- und Release Liner Folien verwendet, die keinen Wirkstoffdurchtritt und nur einen geringen Gasaustausch erlauben.

Dass die Release-Liner-Folie und die Trägerschicht voneinander lösbar aneinander gebunden sind, bedeutet, dass die Bindung zwischen den beiden Schichten so stark ist, dass die Release-Liner-Folie an der Trägerschicht haftet und an dieser nach der Produktion und während Transport und Lagerung entlang der umfangseitigen Trennkante über einen Siegelrand gebunden ist, dass die beiden Schichten jedoch bei einer herkömmlichen Verwendung des Pflasters durch Abziehen der Release-Liner-Folie voneinander gelöst werden können, d.h., dass weder die Release-Liner-Folie noch die Trägerschicht beim Abziehen der Release-Liner-Folie von der Trägerschicht zerstört werden. Zu diesem Zweck werden der von den zur thermischen Bewirkung verwendeten Werkzeugen ausgeübte Druck sowie die Breite des durch die thermische Bewirkung entstehenden Siegelrandes in Abhängigkeit von den jeweiligen Verhältnissen im Pflaster, wie Dicke und Material der Trägerschicht, Matrix und Release-Linerschicht eingestellt. Beispielsweise kann der die Release-Liner-Folie und die Trägerschicht verbindende Siegelrand eine Breite von 50-1000 µm, insbesondere von 100-300 µm haben.

Um bei der thermischen Bewirkung der umfangseitigen Schnittkante eine Verdrängung der Wirkstoffmatrix aus dem Bereich des Siegelrands zu ermöglichen, umfasst die Wirkstoffmatrix vorteilhafterweise ein Polymer, das günstige Fließeigenschaften und eine niedrige Oberflächenspannung hat. Silikonbasierte Polymere, insbesondere drucksensitive Polyorganosiloxankleber, wie z.B. Dimethylsiloxane, erfüllen diese Bedingungen in besonderem Maße, da sie sich auf Grund ihrer vikoelastischen Eigenschaften leicht aus dem Bereich des Siegelrands verdrängen lassen. Dies ist insbesondere in einer bevorzugten Ausführungsform der Fall, wenn die Materialien der Release-Liner-Folie und Trägerschicht silikonisiert sind. Die Wirkstoffschicht hat dabei bevorzugt eine Dicke von 35-120 µm, besonders bevorzugt von 40-80 µm und ganz besonders bevorzugt von 45-60µm. In einer bevorzugten Ausführungsform der Erfindung erfolgt die Verdrängung der Wirkstoffmatrix aus der vorgesehenen umfangseitigen Trennkante während des thermischen Vereinzelungsschrittes. Denkbar ist allerdings auch, daß in einem ersten Schritt eine Trennkantenkontur durch Druckeinwirkung vorgegeben wird, und in einem zweiten Schritt die thermisch bewirkte Vereinzelung und Versiegelung an der vorbewirkten Trennkantenkantur erfolgt. Dieses zweistufige Verfahren kann insbesondere von Vorteil sein, wenn der Wirkstoff im Pflaster thermisch labil ist und vorab aus dem thermisch zu bewirkenden Schnittkantenbereich verdrängt werden soll und/oder wenn die Wirkstoffmatrix sich in einem einzigen Verarbeitungsschritt nur schwer aus dem Bereich des vorgesehenen Siegelrandes verdrängen lässt.

In den hydrophoben, vorzugsweise silikonbasierten Klebern können neben dem Wirkstoff noch andere Hilfsstoffe, wie z.B. Antioxidanzien wie Natriumbisulfit oder alpha-Tocopherol, Penetrationsbeschleuniger oder Kristallisationsinhibitoren wie Polyvinylpyrrolidon (Kollidon^{®}, Bayer AG), Polyethylenglycol, Polypropylenglycol, Glycerol und Glycerol-Fettsäureester, sowie Copolymere aus Vinylacetat mit Ethylen oder PVP enthalten sein.

Mit Vorteil weist zumindest die Release-Liner-Folie als Sollbruchkante einen geschwächten und/oder zumindest teilweise unterbrochenen Bereich auf, der insbesondere als Öffnungshilfe für das Pflaster verwendbar ist. Eine derartige Sollbruch-Trennkante dient dem Trennen der Release-Liner-Folie in zwei Teile während der Benutzung des Pflasters entlang einer definierten Kante. Hierfür kann die Release-Liner-Folie geschwächt sein, ohne dass eine Versiegelung der Wirkstoffschicht nach außen im Fall einer thermischen Bindung zwischen der Trägerschicht und der Release-Liner-Folie dadurch gestört wird. Eine teilweise Unterbrechung der Release-Liner-Folie entlang der Sollbruch-Trennkante der Release-Liner-Folie kann dabei derart bemessen sein, dass die Versiegelungswirkung der Wirkstoffschicht kontrolliert bleibt. Der geschwächte und/oder zumindest teilweise unterbrochene Bereich bildet dabei eine Sollbruch-Trennkante, die bevorzugt keinen umfangseitigen Rand des Pflasters definiert, sondern die im Allgemeinen entlang eines Wegs auf dem Pflaster verläuft. Die Sollbruch-Trennkante verläuft dabei typischerweise in der Ausdehnungsebene des Pflasters entlang einer Gerade.

In einer bevorzugten Ausführungsform sind die Release-Liner-Folie und die Trägerschicht aus demselben Material gefertigt. Beispiele für solche Materialien sind Polyethylen, Polypropylen sowie Polyethylenterephthalat. Ein Vorteil der Fertigung der Release-Liner-Folie und der Trägerschicht aus demselben Material besteht beispielsweise darin, dass die thermischen Eigenschaften hinsichtlich ihrer Festigkeit sowie die Dichtungseigenschaften der Release-Liner-Folie und der Trägerschicht, abgesehen von möglicherweise unterschiedlichen Stärken der einzelnen Schichten, gleich sind. Dies führt zu einer vereinfachten Verarbeitbarkeit, insbesondere kann für beide Schichten eine thermisch bewirkte umfangseitige Trennkante erzeugt werden, die für beide Schichten gleich verläuft. Vorzugsweise werden Release-Liner-Folie und Trägerschicht aus einem Material gefertigt, dessen Schmelztemperatur in einem Temperaturbereich liegt, bei dem der im Pflaster enthaltene Wirkstoffs noch keine thermischchemische Zersetzung oder Umwandlung eingeht.

In einer bevorzugten Ausführungsform weist die Release-Liner-Folie und/oder die Trägerschicht eine vordefinierte Stärke oder Dicke zur Steuerung eines Gasdurchflusses durch diese Schicht auf. Je nach Material, aus dem die Release-Liner-Folie und/oder die Trägerschicht hergestellt sind, kann jede dieser Schichten einen gewissen Gasaustausch zwischen der Wirkstoffschicht und der Umgebung ermöglichen. Typische Stärken der Release-Liner-Folie liegen bei etwa 50-150 µm und der Trägerschicht zwischen etwa 15 µm und etwa 30 µm. Eine größere Stärke der Schichten führt dabei zu einem verringerten Gasaustausch. Bei der Wahl der Stärke der Schicht ist zur Regelung des Gasdurchflusses auch eine Porosität des Materials der jeweiligen Schicht zu beachten, die auch bevorzugt vordefiniert und besonders bevorzugt für die Release-Liner-Folie und Trägerschicht gleich ist. Daneben ist es auch denkbar, dass das Material der Trägerschicht und/oder der Release-Liner-Folie auf ein gewünschtes Maß eines Gasdurchflusses angepasst wird.

Bei einem erfindungsgemäßen Verfahren zur Herstellung eines transdermalen Pflasters ist zwischen einer Release-Liner-Folie und einer Trägerschicht eine Wirkstoffschicht z.B. mit Rotigotin und/oder einem seiner pharmakologisch akzeptablen Salze als Wirkstoff angeordnet, wobei die Release-Liner-Folie und/oder die Trägerschicht des Pflasters mit zumindest einer thermisch bewirkten umfangseitigen Trennkante versehen wird, um Kristallisation im Bereich dieser Trennkante zu verhindern. Bevorzugt wird die Release-Liner-Folie und/oder die Trägerschicht des Pflasters mit mehreren umfangseitigen und über ihre gesamte Länge thermisch bewirkten Trennkanten versehen.

"Und/oder" heißt wiederum, dass mindestens eine der Release-Liner-Folie und der Trägerschicht des Pflasters mit zumindest einer thermisch bewirkten umfangseitigen Trennkante versehen wird. Dadurch, dass die Trennkante thermisch bewirkt wird, können sowohl Kristallisationskeime als auch andersartige Keime unterbunden bzw. abgetötet werden. Somit kann insbesondere die Entstehung von Kristallen bereits bei der Herstellung des Pflasters verhindert werden.

Bei einem bevorzugten Verfahren werden die Release-Liner-Folie und die Trägerschicht entlang der umfangseitigen Trennkanten über eine Länge von mindestens 70% der umfangseitigen Trennkanten, bevorzugt über die gesamte Länge der umfangseitigen Trennkanten gleichzeitig thermisch miteinander verbunden. Dies geschieht insbesondere derart, dass die Wirkstoffschicht durch die thermisch miteinander verbundenen Schichten versiegelt ist. Bevorzugt ist es also möglich, dass die umfangseitige Trennkante gleichzeitig einen Siegelrand zwischen der Trägerschicht und der Release-Liner-Folie bildet. Das Verbinden der Release-Liner-Folie mit der Trägerschicht entlang der umfangseitige Trennkante erfolgt dabei im Wesentlichen während desselben Vorgangs, der auch die umfangseitige Trennkante selbst realisiert. Beispielsweise ist es möglich, dass durch Verwendung eines Paars gegenläufiger, beheizbarer Walzen oder ähnlicher Werkzeuge einerseits eine umfangseitige Trennkante thermisch bewirkt und andererseits die Release-Liner-Folie mit der Trägerschicht thermisch miteinander verbunden wird. Die Release-Liner-Folie und die Trägerschicht werden derart miteinander verbunden, dass sie danach voneinander lösbar sind, d.h., dass die Release-Liner-Folie von der Trägerschicht ablösbar ist, ohne dass eine der beiden Schichten dabei zerstört werden muss.

Bevorzugt wird die Release-Liner-Folie in einem Bereich einer Sollbruchkante, die insbesondere als Öffnungshilfe für das Pflaster verwendbar ist, thermisch geschwächt und/oder zumindest teilweise unterbrochen. Die Sollbruch-Trennkante, die als Öffnungshilfe für das Pflaster verwendbar ist, liegt im Inneren des Pflasters, d.h., innerhalb des umlaufenden Randes, der durch eine thermisch bewirkte umfangseitige Trennkante gebildet werden kann. Bei der thermisch geschwächten und/oder zumindest teilweise unterbrochenen Sollbruch-Trennkante kann es sich beispielsweise um eine Öffnungshilfe entsprechend dem so genannten S-Cut handeln.

Dabei wird zumindest eine der thermischen Behandlungen oberhalb einer Schmelztemperatur der jeweiligen Wirkstoffkristalle durchgeführt. Die thermischen Behandlungen bezeichnen dabei einerseits das Versehen des Pflasters mit zumindest einer thermisch bewirkten Trennkante und andererseits das Verbinden der Release-Liner-Folie mit der Trägerschicht entlang der umfangseitigen Trennkante. Im Beispiel von Rotigotin als Wirkstoff in der Wirkstoffschicht liegt der Schmelzpunkt des entsprechenden höherschmelzenden Rotigotinkristalls ("Form II") bei 97°C ± 2°C. Um die Schneidwerkzeuge kristallkeimfrei zu halten wird daher bevorzugt, diese auf eine höhere Temperatur als den Schmelzpunkt des jeweiligen Wirkstoffkristalls, beispielsweise also auf mindestens 100°C, vorzugsweise auf über 110 °C oder über 120°C im Fall von Rotigotin zu bringen.

Zudem muß die Temperatur so hoch gewählt werden, dass Sie eine thermische Bewirkung der Schnittkanten und bevorzugt ein Verschmelzen der Release-Liner-Folie mit der Trägerschicht gewährleistet. Die hierfür erforderlichen Temperaturen hängen von der Art der für die Release-Liner-Folie und die Trägerschicht verwendeten Materialien ab und betragen z.B. für Polyethylenfolien ca 100-120°C und mehr, für Polypropylenfolien etwa 165°C und mehr sowie für Polyethylenterephtalat ca 260°C und mehr. Dabei werden die Materialien der Release-Liner-Folie und der Trägerschicht bevorzugt so gewählt, dass die Schmelztemperaturen besagter Folien unterhalb der Zersetzungstemperatur des jeweils im Pflaster vorliegenden Wirkstoffes liegen.

Mit Vorteil sind die Release-Liner-Folie und/oder die Trägerschicht mit der Wirkstoffschicht versehen und die Schichten werden mit der Wirkstoffschicht durch ein Paar beheizbare gegenläufige Walzen, die als Walzenschleuse fungieren, durchgeführt. Bei diesem Verfahren könnte die Pflasteroberfläche auf der Oberfläche einer der gegenläufigen Walzen als beheizbare Struktur ausgeprägt sein. Eine der beiden Walzen kann dazu dienen, umfangseitige Trennkanten zu erzeugen, insbesondere einzelne Pflaster aus den Schichten zu vereinzeln. Dagegen kann die andere der beiden Walzen die Struktur der Sollbruch-Trennkante, z.B. in Form eines "S-Cuts" oder einer entsprechenden, ähnlichen Öffnungshilfe tragen. Hierbei ist es nicht unbedingt notwendig, dass beide Walzen beheizbar sind. Es kann auch die Walze zur Erzeugung einer thermisch bewirkten umfangseitigen Trennkante beheizt sein, während die andere Walze zur Erzeugung der Struktur der Sollbruch-Trennkante (z.B. des S-Cuts) nur eine gezielte Schwächung der Release-Liner-Folie bewirken soll und nicht beheizbar sein muss, wenn sie nicht mit der Wirkstoffschicht in Kontakt kommt. Bevorzugt ist jedoch, dass sowohl eine Walze zur Erzeugung einer umfangseitigen Trennkante als Rand des Pflasters als auch eine Walze zur Erzeugung einer Sollbruch-Trennkante mit einem geschwächten und/oder zumindest teilweise unterbrochenen Bereich, insbesondere als Öffnungshilfe für das Pflaster, beheizbar sind. Es ist schließlich auch möglich, dass sowohl eine umfangseitige Trennkante als Rand des Pflasters als auch eine Sollbruch-Trennkante mit geschwächtem und/oder zumindest teilweise unterbrochenem Bereich durch eine einzige Walze, die beheizbar ist, bewirkt werden, während eine gegenläufige Gegenwalze unbeheizt sein kann.

Ein weiterer Gegenstand der Erfindung ist ein Verfahren zur Herstellung eines transdermalen Pflasters, wobei zwischen einer Release-Liner-Folie und einer Trägerschicht eine Wirkstoffschicht mit einem schlecht wasserlöslichen Wirkstoff, insbesondere mit Rotigotin und/oder einem seiner pharmakologisch akzeptablen Salze als Wirkstoff, ganz besonders bevorzugt mit der freien Base von Rotigotin angeordnet ist, wobei das Verfahren dadurch charakterisiert ist, dass unmittelbar bevor die Release-Liner-Folie und/oder die Trägerschicht mit einer umfangseitigen Trennkante versehen wird, die Release-Liner-Folie und/oder die Trägerschicht elektrisch entladen wird bzw. werden. Durch das Versehen der Release-Liner-Folie und/oder der Trägerschicht mit der thermisch bewirkten umfangseitigen Trennkante kann dabei insbesondere eine Vereinzelung der Pflaster durchgeführt werden. Durch das elektrische Entladen der Release-Liner-Folie und/oder der Trägerschicht ist eine weitere Reduktion des Risikos der Wirkstoffkristallisation in der Wirkstoffschicht möglich. Der Ausdruck "unmittelbar bevor die Release-Liner-Folie und/oder die Trägerschicht mit einer umfangseitigen Trennkante versehen wird" bedeutet dabei, dass auf die entsprechende Schicht zwischen dem elektrischen Entladen und dem Versehen mit einer umfangseitigen Trennkante kein weiterer Verfahrensschritt angewendet wird und dass diese Schicht zwischen dem Entladen und dem Versehen mit einer umfangseitigen Trennkante keine unnötige Wegstrecke zurücklegt. Dies hat den Vorteil, dass zwischen dem Entladen und dem Versehen der Schicht mit einer umfangseitigen Trennkante kein erneutes Aufladen einer der Schichten erfolgen kann. Für die Entladung der Release-Liner-Folie und/oder der Trägerschicht unmittelbar vor der Vereinzelung bzw. dem Versehen mit einer umfangseitigen Trennkante kann das Abführen der Spannung beispielsweise über eine großflächige elektrische Seite erfolgen. Die Spannung kann zum Beispiel über Carbonfäden oder Edelstahldrähte abgeführt werden. Die Spannungsableitung ist dabei bevorzugt mit dem thermischen Vereinzelungsverfahren kombiniert. In einer bevorzugten Ausführungsform werden daher Release-Liner-Folie und Trägerschicht beide jeweils elektrisch entladen und sodann jeweils mit einer thermisch bewirkten umfangseitigen Trennkante versehen und voneinander lösbar aneinander gebunden, so dass die zwischenliegende wirkstoffhaltige Schicht versiegelt ist.

Bei einem ferner bevorzugten Verfahren zur Herstellung eines transdermalen Rotigotinpflasters liegt der Wirkstoff in amorpher Form vor. Mit Vorteil liegt Rotigotin in der Wirkstoffschicht im Wesentlichen, d.h zu über 90 Mol% in Form der Wirkstoffbase und in 5-20 Gew% bezogen auf das Gewicht der Wirkstoffschicht vor. In dem somit hergestellten Rotigotinflaster sind auch nach einer Lagerung von 12 Monaten bei 25°C, bevorzugt bei 24 Monaten bei 25°C keine Kristalle von Rotigotin Form II nachweisbar. Dass der Wirkstoff in amorpher Form vorliegt bedeutet, dass er im Wesentlichen keine Kristallstrukturen aufweist.

Das erfindungsgemäße Verfahren dient zur Herstellung eines transdermalen Pflasters, bei dem zwischen einer Release-Liner-Folie und einer Trägerschicht eine Wirkstoffschicht angeordnet ist, die einen amorphen, schwer wasserlöslichen Wirkstoff, insbesondere Rotigotin oder eines seiner pharmakologisch akzeptablen Salze enthält und im Wesentlichen frei ist von Kristallisationskeimen des Wirkstoffs. Die Release-Liner-Folie und/oder die Trägerschicht des Pflasters werden dabei mit zumindest einer thermisch bewirkten Trennkante versehen. Der Ausdruck "im Wesentlichen" bringt dabei zum Ausdruck, dass Kristallstrukturen bis zu einem Anteil von weniger als 1 Gew% bezogen auf den jeweils eingesetzten Wirkstoffgehalt vorhanden sein dürfen, ohne, dass der Wirkstoff als in zumindest teilweise kristalliner Form vorliegend bezeichnet wird.

Gegenstand der Erfindung ist auch die Verwendung eines oben beschriebenen Verfahrens zur Unterdrückung des Auftretens von Rotigotinkristallen im Pflaster.

Gegenstand der Erfindung ist ferner ein Werkzeug zum Herstellen eines transdermalen Pflasters gemäß obiger Beschreibung durch das oben beschriebene Verfahren.

Das Werkzeug umfasst zwei gegenläufige, zumindest teilweise beheizbare Walzen, durch welche die Release-Liner-Folie und die Trägerschicht sowie die Wirkstoffschicht durchführbar sind. Die Wirkstoffschicht kann dabei auf der Release-Liner-Folie und/oder der Trägerschicht aufgebracht sein. Der Ausdruck "durchführbar" bedeutet hierbei, dass die entsprechende Schicht zwischen den zwei gegenläufigen Walzen, die eine Walzenschleuse bilden, hindurchgeführt werden kann.

Zumindest eine der beiden Walzen ist dabei zumindest teilweise beheizbar, um zumindest eine Trennkante des Pflasters thermisch zu bewirken. Dabei ist es denkbar, dass die Walze nur im Bereich eines Werkzeugelements beheizbar ist, welches zur Ausprägung einer umfangseitigen Trennkante geeignet ist. Die Geometrie des zur Ausprägung der umfangseitigen Trennkante geeigneten Werkzeugs, das als Siegelelement zur Versiegelung der Wirkstoffschicht wirkt ist bevorzugt derart gewählt, dass die Flussrichtung der Wirkstoffhaltigen Matrix ins Pflasterinnere gerichtet ist. Diese bevorzugte Ausführung des Siegelelements erlaubt längere Reinigungsintervalle, eine gegebenenfalls längere Wartungsfreiheit und/oder Lebensdauer des Werkzeugs. Neben der Ausführung des Werkzeugs mit nur einem beheizbaren Werkzeugelement ist es jedoch auch möglich, dass eine Walze vollständig beheizbar ist oder dass beide Walzen teilweise bzw. vollständig beheizbar sind. Die entsprechenden Teile der Walze oder Walzen sind dabei so konstruiert, dass sie, beispielsweise durch einen elektrischen Widerstand, auf eine gewünschte Temperatur gebracht werden können, die bevorzugt oberhalb einer Schmelztemperatur eines Wirkstoffkristalls und/oder oberhalb der Sterilisationstemperatur eines bestimmten Keims liegen kann. Im Fall von Rotigotin ist es von Vorteil, wenn wenigstens die Walze, die zur Vereinzelung des Pflasters ausgebildet ist, auf mindestens 100°C, oder auf über 110°C oder über 120°C erhitzbar ist. Wenn Polypropylen bzw Polyethylenterephtalat-Folien in der Release-Liner- und/oder Trägerschicht zur Anwendung kommen, ist eine Erhitzung der zur Einführung der Trennkanten verwendeten Werkzeugelemente auf ca 170°C bzw. ca. 260°C und darüber vorteilhaft.
Darüber hinaus ist es möglich, dass mehr als zwei Walzen zur Durchführung des oben beschriebenen Verfahrens zur Herstellung eines oben beschriebenen Pflasters eingesetzt werden, zum Beispiel in Form einer sequentiellen Anordnung mehrerer zueinander gegenläufiger Walzenpaare.

In einer bevorzugten Ausführungsform ist eine erste Walze zur Vereinzelung des Pflasters durch zumindest eine umfangseitige Trennkante eingerichtet, und eine zweite, zur ersten gegenläufige Walze zur Ausbildung eines geschwächten und/oder zumindest teilweise unterbrochenen Bereichs durch eine Sollbruchkante eingerichtet. Wie oben beschrieben, wird die Vereinzelung des Pflasters durch eine thermisch bewirkte umfangseitige Trennkante vollzogen, welche die Schichten durchtrennt. Die Ausbildung des geschwächten und/oder zumindest teilweise unterbrochenen Bereichs als Sollbruch-Trennkante erfolgt dagegen bevorzugt derart, dass lediglich die Release-Liner-Folie geschwächt und/oder zumindest teilweise unterbrochen wird, also nicht alle Schichten durchtrennt werden. Aus diesem Grund kann die zweite, zur Herstellung der Sollbruchkante ausgebildete Walze auch derart ausgestaltet sein, dass sie die Sollbruchkante nicht thermisch sondern rein mechanisch beispielsweise durch Schnitte bewirkt. In einer bevorzugten Ausführungsform der Erfindung ist allerdings auch die zweite Walze beheizbar.

Neben derartigen, bevorzugten Walzen kann das Werkzeug auch durch eine Stanzvorrichtung mit einem erhitzten Stanzwerkzeug gegeben sein.

In einer weiteren bevorzugten Ausführungsform ist das Werkzeug dazu geeignet, eine Vielzahl gleichartiger transdermaler Pflaster ausgehend von jeweils einer Bahn einer Release-Liner-Folie, einer Trägerschicht und einer Wirkstoffschicht zu vereinzeln. Dieses Werkzeug dient also einer Herstellung von transdermalen Pflastern im industriellen Maßstab, bei der die Trennkanten thermisch bewirkt werden. Durch die thermische Erzeugung der Trennkanten wird die Entstehung von Keimen, insbesondere von Kristallkeimen direkt bei der Herstellung verhindert.

Gegenstand der Erfindung ist zudem die Verwendung eines erfindungsgemäßen Werkzeugs bei der Durchführung des erfindungsgemäßen Verfahrens zur Herstellung eines erfindungsgemäßen transdermalen Pflasters.

Bei einem oben beschriebenen transdermalen Pflaster werden ein Kristallwachstum begünstigende Einflüsse der Atmosphäre an den Pflasterkanten, beispielsweise durch Sauerstoffeintrag, Wasseraufnahme, Verdunstungsverluste oder Kontamination der Produktionsstätte durch einzelne "Impf"kristalle der "Form II" von Rotigotin bzw. anderer Wirkstoffkristalle gezielt verhindert.

Beim erfindungsgemäßen Verfahren wird eine Kontamination des Trennwerkzeugs mit einem Wirkstoffkristall, beispielsweise mit Rotigotin "Form II", und folglich eine sich ansonsten anschließende Kontamination der Trennkante, die durch das Trennwerkzeug erzeugt wird, unterbunden. Auch mechanische Scherkräfte an den mechanisch bewirkten Trennkanten und/oder elektromechanische Impulse beim Vereinzeln der Pflaster, die durch Spannungsunterschiede zwischen der Release-Liner-Folie und der Trägerschicht oder andere Effekte und anschließenden Kurzschluss beim mechanischen Vereinzeln hervorgerufen werden, werden bei einem bevorzugten Verfahren zuverlässig verhindert. Schließlich wird eine Kristallkeimbildung an Oberflächendefekten mechanisch bewirkter Trennkanten vermieden.

Es wird folglich ein transdermales Pflaster zur Verfügung gestellt, das die Entstehung von Kristallen in der Wirkstoffschicht des Pflasters bereits in seiner Herstellung verhindert bzw. deutlich reduziert.

Die erfindungegemäßen transdermalen Pflaster sind zur Behandlung von Erkrankungen geeignet. Ist Rotigotin oder eines seiner pharmazeutisch akzeptablen Salze als Wirkstoff im Pflaster enthalten, sind die transdermalen Pflaster insbesondere zur Behandlung von Erkrankungen geeignet, die mit Störungen des Dopaminstoffwechsels und/oder Störungen der dopaminergen Signalkaskade einhergehen. Rotigotin ist daher insbesondere geeignet zur Behandlung und/oder Prävention einer Erkrankung ausgewählt aus der Gruppe
- Parkinson Plus,
- Depression,
- Fibromyalgie, sowie insbesondere
- Morbus Parkinson und
- Restless Leg Syndrom.

Ein weiterer Gegenstand der Erfindung ist daher die Verwendung eines vorstehend sowie in den Ansprüchen beschriebenen Pflasters, wobei der Wirkstoff Rotigotin oder eines seiner pharmakologisch akzeptablen Salze, und insbesondere die freie Base von Rotigotin ist, zur Herstellung eines Arzneimittels zur Prävention und/oder Behandlung einer Erkrankung ausgewählt aus Parkinson Plus, Depression, Fibromyalgie sowie bevorzugt Morbus Parkinson und Restless Leg Syndrom.

Weitere Vorteile und bevorzugte Ausführungsformen ergeben sich aus der Gesamtheit der Ansprüche und der nachfolgenden Beispielbeschreibung.

### Kurze Figurenbeschreibung

- Fig. 1: zeigt ein Foto eines transdermalen Pflasters aus dem Stand der Technik, bei dem entlang einer Trennkante Kristallwachstum in der Wirkstoffschicht zu beobachten ist;
- Fig. 2: zeigt ein Foto eines erfindungsgemäßen transdermalen Pflasters mit einer thermisch bewirkten Trennkante ohne Kristallwachstum in der Wirkstoffschicht; und
- Fig. 3: zeigt ein Foto eines transdermalen Pflasters, das gemäß Vergleichsbeispiel 1 mit einer Trennkante versehen wurde und Kristallwachstum aufweist.

### Beispiele

### Beispiel 1

Aus einem Rotigotin-haltigen Pflaster, bei dem an den Schnitträndern bereits Kristallwachstum zu beobachten war, wurde aus einem kristallfreien Abschnitt mit einem Hitzedraht ein kleineres Pflaster ausgeschnitten. Der Hitzedraht war dabei Teil einer Styroporschneidemaschine. Dabei wurden die gegenüberliegenden Flächen des Pflasters, also die Release-Liner-Folie und die Trägerschicht thermisch miteinander verklebt. Das Pflaster wurde bei unterschiedlichen Temperaturen, nämlich 1 Woche bei ca. 4°C und anschließend ca. 10 Monate bei Raumtemperatur gelagert. Diese thermisch bewirkte Trennkante des Pflasters zeigte nach einer optischen Inspektion der Kante kein Kristallwachstum.

### Beispiel 2

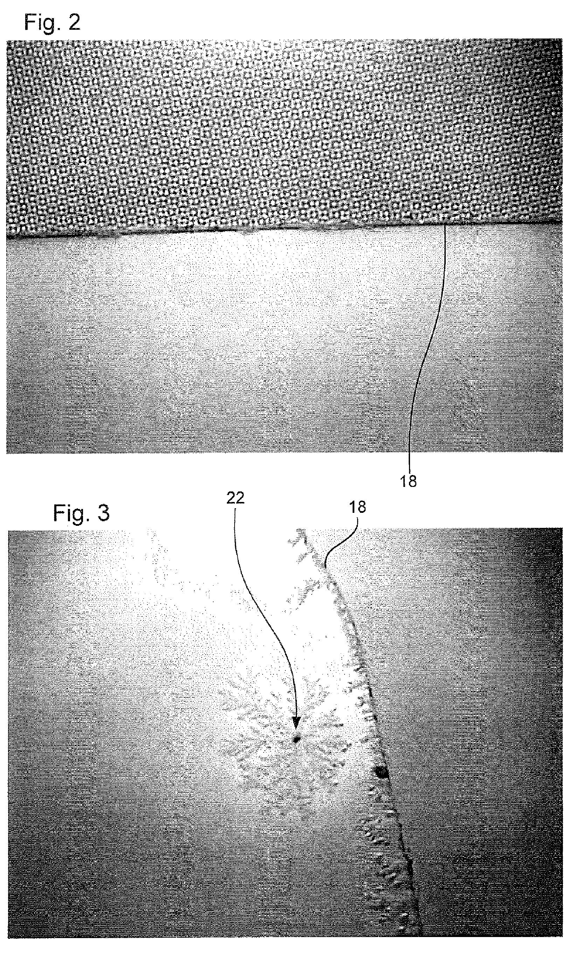

Aus einem Rotigotin-haltigen Pflaster, bei dem an den Schnitträndern bereits Kristallwachstum zu beobachten war, wurde aus einem kristallfreien Abschnitt mit einem handelsüblichen Folienschweißgerät ein kleineres Pflaster ausgelöst. Dabei wurden die gegenüberliegenden Flächen des Pflasters, also die Release-Liner-Folie und die Trägerschicht, thermisch miteinander verklebt. Das Pflaster wurde bei unterschiedlichen Temperaturen, nämlich eine Woche bei ca. 4°C und anschließend ca. 10 Monate bei Raumtemperatur gelagert. Fig. 2 zeigt ein Foto des so behandelten Pflasters in 10-facher Vergrößerung nach der oben beschriebenen Lagerung. Es ist deutlich zu erkennen, dass die optische Inspektion kein Kristallwachstum an der Trennkante 18 erkennen lässt.

### Vergleichsbeispiel 1

In einem kristallfreien Abschnitt eines Rotigotin-haltigen Pflasters wurde mit einer Haushaltsschere eine neue Trennkante erzeugt. Die Umgebung dieser Trennkante wurde mit einem Hochspannungsgenerator elektrischen Feldern, nämlich ca. 10.000 V ausgesetzt, um den Einfluss elektrischer Ladungen auf den Schichten auf das Kristallwachstum zu erforschen. Das Pflaster wurde bei Raumtemperatur für zwei Wochen gelagert. Wie Fig. 3 zu entnehmen ist, die ein Foto dieser Trennkante zeigt, ist ein deutlich sichtbares Kristallwachstum an der Trennkante 18 sowie an der der Funkenstrecke ausgesetzten Stelle 22 zu beobachten.

## Patentansprüche

1. Transdermales Pflaster, umfassend
eine Release-Liner-Folie,
eine wirkstoffhaltige Polymerschicht mit einem amorphen Wirkstoff, und
eine Trägerschicht,
wobei die Wirkstoffschicht zwischen der Release-Liner-Folie und der Trägerschicht eingebracht ist, wobei
die Release-Liner-Folie und/oder die Trägerschicht über eine Länge von mindestens 70%, bevorzugt über die gesamte Länge der umfangseitigen Trennkanten, thermisch und voneinander lösbar derart aneinander gebunden sind, dass die Wirkstoffschicht **dadurch** versiegelt ist.

2. Transdermales Pflaster nach Anspruch 1, wobei der Wirkstoff Rotigotin oder eines seiner pharmakologisch akzeptablen Salze, und insbesondere die freie Base von Rotigotin ist.

3. Transdermales Pflaster nach einem der vorstehenden Ansprüche, wobei die wirkstoffhaltige Polymerschicht im Wesentlichen aus einem silikonbasierten Kleber besteht, in dem der Wirkstoff in amorpher Form vorliegt.

4. Transdermales Pflaster nach einem der vorstehenden Ansprüche, bei dem zumindest die Release-Liner-Folie als Sollbruchkante einen geschwächten und/oder zumindest teilweise unterbrochenen Bereich aufweist, der insbesondere als Öffnungshilfe für das Pflaster verwendbar ist.

5. Transdermales Pflaster nach einem der vorstehenden Ansprüche, bei dem die Release-Liner-Folie und die Trägerschicht aus demselben Material gefertigt sind.

6. Transdermales Pflaster nach einem der vorstehenden Ansprüche, bei dem die Release-Liner-Folie und/oder die Trägerschicht eine vordefinierte Stärke zur Steuerung eines Gasdurchflusses durch diese Schicht aufweist/aufweisen.

7. Transdermales Pflaster nach einem der vorstehenden Ansprüche, wobei Rotigotin in der Wirkstoffschicht im Wesentlichen in Form der Wirkstoffbase und in 5-20 Gew% bezogen auf das Gewicht der Wirkstoffschicht vorliegt.

8. Transdermales Pflaster nach Anspruch 7 zur Behandlung und/oder Prävention einer Erkrankung ausgewählt aus Morbus Parkinson, Restless Leg Syndrom, Parkinson Plus, Depression, und Fibromyalgie.

9. Verwendung eines Pflasters nach einem der Ansprüche 1-7, wobei der Wirkstoff Rotigotin oder eines seiner pharmakologisch akzeptablen Salze, und insbesondere die freie Base von Rotigotin ist, zur Herstellung eines Arzneimittels zur Behandlung und/oder Prävention einer Erkrankung ausgewählt aus Parkinson Plus, Depression, Fibromyalgie sowie insbesondere Morbus Parkinson und Restless Leg Syndrom.

10. Verfahren zur Herstellung eines transdermalen Pflasters, wobei zwischen einer Release-Liner-Folie und einer Trägerschicht eine Wirkstoffschicht mit Rotigotin und/oder einem seiner pharmakologisch akzeptablen Salze als Wirkstoff angeordnet ist,
wobei die Release-Liner-Folie und/oder die Trägerschicht des Pflasters mit zumindest einer thermisch bewirkten umfangseitigen Trennkante, bevorzugt mit über ihre gesamte Länge thermisch bewirkten umfangseitigen Trennkanten versehen wird, und
wobei die Release-Liner-Folie und die Trägerschicht entlang der umfangseitigen Trennkanten über eine Länge von mindestens 70% der umfangseitigen Trennkanten, bevorzugt über die gesamte Länge der umfangseitigen Trennkanten gleichzeitig thermisch miteinander verbunden werden, insbesondere derart, dass die Wirkstoffschicht durch die thermisch miteinander verbundenen Schichten versiegelt ist.

11. Verfahren nach Anspruch 10, wobei die Release-Liner-Folie in einem Bereich einer Sollbruchkante, die insbesondere als Öffnungshilfe für das Pflaster verwendbar ist, thermisch geschwächt und/oder zumindest teilweise unterbrochen wird.

12. Verfahren nach einem der Ansprüche 10 bis 11, wobei zumindest eine der thermischen Behandlungen oberhalb einer Schmelztemperatur eines Wirkstoffkristalls, bevorzugt oberhalb einer Temperatur von 100°C, besonders bevorzugt oberhalb von 120°C durchgeführt wird.

13. Verfahren nach einem der Ansprüche 10 bis 12, wobei die Release-Liner-Folie und/oder die Trägerschicht mit der Wirkstoffschicht versehen sind und wobei die Schichten mit der Wirkstoffschicht durch ein Paar beheizbare gegenläufige Walzen durchgeführt werden.

14. Verfahren nach einem der Ansprüche 10 bis 13, wobei unmittelbar bevor die Release-Liner-Folie und/oder die Trägerschicht mit einer thermisch bewirkten Trennkante versehen wird, die Release-Liner-Folie und/oder die Trägerschicht elektrisch entladen wird.

15. Verfahren nach einem der Ansprüche 10 bis 14 zur Herstellung eines transdermalen Rotigotinpflasters, wobei der Wirkstoff in amorpher Form vorliegt und in dem auch nach einer Lagerung von 12 Monaten bei 25°C, bevorzugt von 24 Monaten bei 25°C keine Kristalle von Rotigotin Form II nachweisbar sind.

16. Verwendung eines Verfahrens nach einem der Ansprüche 10 bis 15 zur Unterdrückung des Auftretens von Rotigotinkristallen im Pflaster.

17. Werkzeug zum Herstellen eines transdermalen Pflasters nach einem der Ansprüche 1 bis 8 durch ein Verfahren nach einem der Ansprüche 10 bis 15, umfassend zwei gegenläufige, zumindest teilweise beheizbare Walzen, durch welche die Release-Liner-Folie und die Trägerschicht sowie die Wirkstoffschicht durchführbar sind.

18. Werkzeug nach Anspruch 17, insbesondere in Verbindung mit Anspruch 4 und 11, wobei eine erste Walze zur Vereinzelung des Pflasters eingerichtet ist und wobei eine zweite, zur ersten gegenläufige Walze zur Ausbildung eines geschwächten und/oder zumindest teilweise unterbrochenen Bereichs eingerichtet ist.
